# EUROPÄISCHE PATENTSCHRIFT

(11) **EP 1 520 177 B1**
(45) Veröffentlichungstag und Bekanntmachung des Hinweises auf die Patenterteilung: **07.05.2014**
(21) Anmeldenummer: 03760636.5
(22) Anmeldetag: 17.06.2003
(51) Int. Cl.: G01N 33/68

(54) **VERFAHREN UND REAGENZ ZUR SPEZIFISCHEN IDENTIFIZIERUNG UND QUANTIFIZIERUNG VON EINEM ODER MEHREREN PROTEINEN IN EINER PROBE**
METHOD AND REAGENT FOR SPECIFICALLY IDENTIFYING AND QUANTIFYING ONE OR MORE PROTEINS IN A SAMPLE
PROCEDE ET REACTIF DESTINES A L'IDENTIFICATION SPECIFIQUE ET A LA QUANTIFICATION D'AU MOINS UNE PROTEINE DANS UN ECHANTILLON

(30) Priorität: 20.06.2002 DE 10227599
(43) Veröffentlichungstag der Anmeldung: 06.04.2005
(73) Patentinhaber: Proteome Factory AG, 12489 Berlin (DE); Humboldt Universität zu Berlin, 10099 Berlin (DE)
(72) Erfinder: KRAUSE, Martin, Dr., 12167 Berlin (DE); SCHELER, Christian, 14167 Berlin (DE); BÖTTGER, Ulrike, 12487 Berlin (DE); WEISSHOFF, Hardy, 13088 Berlin (DE); LINSCHEID, Michael, 12203 Berlin (DE)
(74) Vertreter: Krauss, Jan
(86) Internationale Anmeldenummer: PCT/EP2003/006391
(87) Internationale Veröffentlichungsnummer: WO 2004/001420

(56) Entgegenhaltungen:
- WO-A-00/47548
- WO-A-00/57183
- US-A- 5 958 783
- US-A- 6 087 452
- T ET AL: "Reversible oriented immobilization of histidine-tagged proteins on gold surfaces using a chelator thioalkane" SUPRAMOLECULAR SCIENCE, OXFORD, GB, Bd. 2, Nr. 3/4, 1995, Seiten 155-160, XP002082734 ISSN: 0958-5677
- ARMISEN P ET AL: "Selective adsorption of poly-His tagged glutaryl acylase on tailor-made metal chelate supports" JOURNAL OF CHROMATOGRAPHY, ELSEVIER SCIENCE PUBLISHERS B.V. AMSTERDAM, NL, Bd. 848, Nr. 1-2, 2. Juli 1999 (1999-07-02), Seiten 61-70, XP004184200 ISSN: 0021-9673
- PORATH J: "IMMOBILIZED METAL ION AFFINITY CHROMATOGRAPHY" PROTEIN EXPRESSION AND PURIFICATION, ACADEMIC PRESS, US, Bd. 3, Nr. 4, August 1992 (1992-08), Seiten 263-281, XP001098444 ISSN: 1046-5928
- GYGI S P ET AL: "QUANTITATIVE ANALYSIS OF COMPLEX PROTEIN MIXTURES USING ISOTOPE-CODED AFFINITY TAGS" NATURE BIOTECHNOLOGY, NATURE PUBLISHING, US, Bd. 17, Nr. 10, Oktober 1999 (1999-10), Seiten 994-999, XP001010578 ISSN: 1087-0156

## Beschreibung

Die vorliegende Erfindung betrifft ein Verfahren und ein zu dessen Durchführung geeignetes Reagenz, das eine reproduzierbare systematische, qualitative und quantitative Proteomcharakterisierung mit Hilfe von nicht-Isotop metallkodierten Markern und u.a. modernsten massenspektrometrischen Tandem-Methoden beinhaltet.

### Hintergrund der Erfindung

Einer der weitreichendsten Erfolge des 20. Jahrhunderts war die Entdeckung der DNS als Träger aller Erbanlagen und die Aufklärung ihrer Eigenschaften und dreidimensionalen Struktur. Die erste komplette DNS-Sequenz eines Organismus, wurde im Jahre 1977 von Fred Sanger publiziert. Seitdem erfuhr die Genom-Forschung einen ungeheuren Aufschwung durch die Entwicklung neuer Technologien und automatisierter Hochdurchsatzverfahren, welche heute die Sequenzierung des kompletten Genoms eines Mikroorganismus zur Routine machen.

Nun steht die Biochemie vor einer neuen, weitaus größeren Herausforderung: die enorme Flut von Genomdaten, die in riesigen elektronischen Bibliotheken gespeichert wird, muß in einen funktionellen Zusammenhang gestellt, der genetische Code in nützliche Information umgewandelt werden. Die Einsicht, daß es unmöglich ist, die Kompliziertheit biologischer Prozesse nur mit Hilfe der Genomanalyse zu klären, hat einen weiteren Wissenschaftszweig der molekularen Zellbiologie auf den Plan gerufen, die Proteom-Forschung. Denn die Genprodukte. d.h. die über die Gene verschlüsselten Proteine, sind die eigentlichen biologischen Effektormoleküle, die in das biologische Geschehen eingreifen, dynamische Prozesse steuern, vielfältige Funktionen ausüben. Erst über sie läßt sich verstehen, wie das menschliche Genom und zelluläre Prozesse funktionieren und auch Krankheiten entstehen.

Als Zweig der Wissenschaft befaßt sich Proteom-Forschung ("Proteomics") mit der systematischen Identifizierung aller Proteine, die in einer Zelle oder einem Gewebe exprimiert wurden und der Charakterisierung ihrer wesentlichen Eigenschaften, wie z.B. Menge, Grad der Modifizierung, Einbindung in Multi-Protein-Komplexe, etc. Es werden Proteindatenbanken bzw. Zellkarten erstellt, in denen Proteinsequenzen archiviert werden. Inzwischen sind viele tausend Sequenzen mit oftmals bekannter Funktion verfügbar.

Zum gegenwärtigen Zeitpunkt erreicht jedoch keine der angewendeten analytischen Proteintechnologien den hohen Durchsatz und das Automatisierungsniveau der Gentechnologie. Es ist weiterhin unwahrscheinlich, daß eine analoge Protein-Vervielfältigung, wie die PCR, in der Proteom-Forschung jemals möglich sein wird. Vielmehr bietet sich hier die Möglichkeit einer Proteinanreicherung an, wobei die interessanten Proteine aufgrund spezifischer Eigenschaften extrahiert bzw. angereichert werden. Z.B. können physikalische Merkmale, wie die Löslichkeit oder die Fähigkeit, an spezifische Liganden zu binden, ausgenutzt werden.

Die Verwendung der Proteomanalyse als schnelle und parallele Methode verglichen mit klassischer Proteinchemie findet in der biologischen Grundlagenforschung, der Biotechnologie und der medizinischen Forschung mehr und mehr Eingang. Es ist damit zu rechnen, daß diese Analytik in wenigen Jahren standardmäßig etabliert sein wird. Wie effektiv eine Proteomanalyse tatsächlich durchgeführt werden kann, hängt in entscheidendem Maße davon ab, wie gut man mit Hilfe der analytischen Methoden in der Lage ist, sogenannte "low-copy" Proteine zu identifizieren und zu quantifizieren, da gerade diese häufig eine entscheidende biologische Rolle im Zellgeschehen spielen.

Die nach wie vor meist angewendete und zuverlässigste Methode der Proteomanalyse ist die zweidimensionale Gelelektrophorese (2DGE), an die sich die sequentielle Identifizierung der getrennten Proteinspezies anschließt. Zu wissenschaftlicher Signifikanz gelangte diese Herangehensweise durch enorme Technologiefortschritte in der Massenspektrometrie und Bioinformatik. Diese erst seit kurzem verfügbare, hochempfindliche MS-Technik hat es ermöglicht, auch geringste Protein- oder Peptidmengen, die mit Hilfe von konventionellen Anfärbemethoden .sichtbar gemacht werden können, im Femtomolbereich zu detektieren und über Tandem-Techniken auch zu identifizieren. Dies sind vor allem die matrixassistierte Laserdesorptions/Ionisations (MALDI) time-of-flight (TOF)-MS und die Elektrospray-ionisations-(ESI)-MS. Tandem-MS-Instrumente wie das Triple-Quadrupole-Gerät, Ion-Trap und das Hybrid Quadrupol-time-of-flight (Q-TOF)-Gerät werden routinemäßig in LC-MS/MS- oder Nanosprayexperimenten mit Elekrospray-Ionisierung (ESI) eingesetzt, um Peptidfragment-Ionenspektren zu erzeugen, die für die Proteinidentifizierung über eine Datenbank-Sequenzsuche geeignet sind.

Die Protein- bzw. Genomdatenbanksuche ist ein ebenso wichtiges Werkzeug, dem die Proteomforschung ihre Fortschritte zu verdanken hat. Die entwickelten Computersuchalgorithmen sind inzwischen sehr ausgefeilt. Goodlett *et al.* konnte zeigen, daß die genaue Masse eines Peptids, zusammen mit einschränkenden Suchkriterien wie dem Molekulargewicht des Proteins, von dem das Peptid stammt und der Angabe der spezifischen Protease zur Spaltung des Proteins, ausreichen kann, um ein Protein durch eine Datenbanksuche eindeutig zu identifizieren. Der hohe Arbeitsaufwand und die oftmals laborübergreifende Nicht-Reproduzierbarkeil der 2DGE-Technik macht es jedoch nahezu unmöglich, diese Methode zu automatisieren. Gegenwärtig gibt es keine, dem Niveau der Genomtechnologie vergleichbare, analytische Technologie auf dem Gebiet der Proteomforschung. Während man mit Hilfe dieser Methoden wohl in der Lage ist, die Komponenten eines Proteingemisches zu analysieren, sind sie leider weder geeignet, die exakte Menge noch den Aktivitätszustand der Proteine in der Mischung zu bestimmen. Ohne einen vorherigen Anreicherungsschritt ist es praktisch nicht möglich, Proteine, die nur in sehr geringen Mengen vorkommen, wie beispielsweise Regulatorproteine, nachzuweisen. Aus diesem Grunde und wegen weiterer bekannter Nachteile der 2DGE sucht man verstärkt nach alternativen Methoden, die eine weitestgehende Unabhängigkeit von der 2DGE als Trennmethode erlauben.

Gelfreie Systeme erregen zunehmend das Interesse der Proteomforscher. Es lassen sich zahlreiche unterschiedliche gelfreie Systeme erdenken, die alle auf der Kombination zweier oder mehrere unterschiedlicher chromatographischer Trennverfahren beruhen. Die chromatographische Trennung von Proteinen ist ein zentraler Bestandteil jeglicher Proteinforschung und somit ein naheliegendes Verfahren in der Proteomforschung. Dank der langjährigen Entwicklung und Optimierung zeichnen sich chromatographische Trennungen durch hohe Reproduzierbarkeit aus. Allerdings hat selbst die Kombination zweier unterschiedlicher chromatographischer Verfahren nicht das in der Proteomforschung benötigte Trennvermögen, da komplexe Proteingemische aufgrund ihrer Eigenschaften schwierig in einzelne aufgereinigte Proteinfraktionen zu trennen sind. Eine Kopplung der Chromatographie an die Massenspektrometrie bringt mit der Massenanalyse ein weitere Trenndimension, die allerdings auf Proteine angewendet nur einen sehr begrenzten Nutzen bringt. Erst bei der Analyse von Peptiden, wie unten aufgeführt, ist dieser Ansatz erfolgversprechend.

In der WO 00/11208 wird eine interessante Alternativmethode für die Proteomanalyse offenbart, die sich besonders für die quantitative Analyse der Proteinexpression in komplexen biologischen Proben, wie Zellen und Geweben, für den Nachweis und die Quantifizierung spezifischer Proteine in komplexen Proteingemischen sowie für die quantitative Bestimmung spezifischer Enzymaktivitäten eignet. Sie bedient sich einer neuen Klasse chemischer Reagenzien, codierten Affinitätstags (CATs), in diesem Fall den sogenannten isotopencodierten Affinitätstags (ICATs) und massenspektrometrischer Methoden.

Das ICAT-Reagenz besteht aus dem Affinitätstag, das selektiv an ein entsprechendes Gegen-Reagenz nichtkovalent bindet und eine säulenchromatographische Trennung der mit dem Affinitätstag markierten Peptide oder Substrate vom übrigen Gemisch erlaubt. Das Affinitätstag ist über einen Linker, der Isotopen-markiert sein kann, mit einer reaktiven Gruppe verknüpft, die selektiv mit einer spezifischen Proteinfunktion reagiert.

Auf diese Weise werden die Proteine, nachdem sie aus den Zellen isoliert wurden, an spezifischen Bindungsstellen durch das ICAT-Reagenz markiert. Hier handelt es sich z.B. um eine funktionelle Gruppe, die eine spezifische Reaktivität für Sulfhydrylgruppen besitzt und ausschließlich an Cystein-haltige Proteine bindet. Aus dem im Anschluß nach enzymatischer Hydrolyse erhaltenen Peptidgemisch werden nur die Cystein-haltigen Peptide selektiv isoliert, dies bedeutet eine wesentliche Vereinfachung der Komplexität des erhaltenen Peptidgemischs, da weniger als ein Zehntel aller Peptide, die beispielsweise durch tryptische Spaltung aus dem gesamten Hefeproteom hervorgegangen sind, einen Cystein-haltigen Rest enthalten. Ein weiterer wesentlicher Vorteil ist, daß auf diesem Wege Proteine, die in nur sehr geringen Mengen auftreten, angereichert werden können. Trotz der starken Vereinfachung der Komplexität des Systems ist aber die Quantifizierung und Identifizierung der Proteine garantiert.

Um relative Mengen an Proteinen in einer oder in mehreren Proteinproben quantitativ zu bestimmen, benutzt man nun unterschiedlich isotopencodierte ICAT-Reagenzien. Jede Probe wird mit einem unterschiedlich isotopenmarkierten, sonst aber chemisch identischen ICAT-Reagenz behandelt. Die Proben, die beispielsweise aus Zellkulturen einer Spezies in unterschiedlichen Entwicklungsphasen stammen können, werden im Anschluß vereinigt und enzymatisch hydrolysiert. Die markierten Peptide werden affinitätschromatographisch vom Gemisch abgetrennt und über HPLC aufgetrennt. Ein Paar identischer, aber aus verschiedenen Proben stammender Peptide wird gleichzeitig von der HPLC-Säule eluiert. Im Massenspektrum besitzen diese Peptide jedoch nicht das gleiche Masse-Ladungs-Verhältnis, sondern sie unterscheiden sich durch die charakteristische Massendifferenz der unterschiedlich isotopenmarkierten Tags. Im Verhältnis der relativen Ionenintensitäten eines solchen (CATmarkierten Peptidpaares im Massenspektrum wird das relative Mengenverhältnis der Stammproteine aus den Ursprungszellen bzw. -gewebe quantitativ widergespiegelt. Mit Hilfe eines Tandem-Massenspektromeiers (MS/MS) wird dann die Peptidsequenz des ICAT- markierten Peptides durch Fragmentierung bestimmt. Die Proteinidentifizierung erfolgt über eine computergestütze Genom- bzw. Proteindatenbanksuche anhand der aufgezeichneten Sequenzinformation.

Trotz aller wesentlichen Vorteile des ICAT-Verfahrens bestehen bei der Durchführung dieses Verfahrens immer noch einige Nachteile, die seine Anwendung im Bereich der Hochdurchsatzanalyse behindern und erschweren. Es müssen Isotope eingesetzt werden, die die Synthesekosten der Verbindungen deutlich erhöhen und nur in begrenztem Maße zu erschwinglichen Preisen erhältlich sind, was die Flexibilität des Verfahrens weiter einschränkt.

Es ist daher Aufgabe der vorliegenden Erfindung, ein verbessertes CAT-basiertes Verfahren zur Verfügung zu stellen, das eine Anwendung von CAT in einer Hochdurchsatz-Umgebung erlaubt. Es ist eine weitere Aufgabe, ein für dieses Verfahren spezifisch geeignetes CAT-Reagenz zur Verfügung zu stellen.

Gemäß einem ersten Aspekt der vorliegenden Erfindung wird diese Aufgabe durch ein Verfahren zur massenspektrometrischen Identifizierung und Quantifizierung von einem oder mehreren Proteinen in einer Probe, die ein Gemisch von Proteinen enthält, gelöst. Das erfindungsgemäße Verfahren umfaßt die Schritte von: a) zur Verfügung stellen einer Probe, die ein Gemisch von Proteinen enthält; b) zur Verfügung stellen eines Reagenz zur Analyse von Peptiden, das die allgemeine Formel

A-Y-PRG

aufweist, worin A mindestens eine funktionelle Gruppe zur reversiblen, kovalenten oder nicht-kovalenten Bindung an ein Trägermaterial darstellt, die mindestens eine Affinitätsfunktion für die selektive Bindung an ein Trägermaterial umfaßt, Y eine Gruppe ist, die mindestens einen makrozyklischen Lanthanoid-Chelatkomplex umfaßt, und PRG eine faßt, und PRG eine reaktive Gruppe zur selektiven Bindung an Peptide oder andere zu analysierende Biomoleküle ist, c) Spaltung der Proteine in der Probe, um Peptide zu erzeugen; d) Kopplung der Peptide mit dem Reagenz aus Schritt b), e) Selektion der in Schritt d) markierten Peptide unter der Verwendung der reversiblen Bindung an ein Trägermaterial oder der Affinitätsmarkierung durch Bindung an ein Trägermaterial und Entfernung von nicht-gebundenen Peptiden, f) Freisetzen der gebundenen Peptide von dem Trägermaterial und Elution von der Matrix; und g) Nachweisen und Identifizieren der markierten Peptide durch Massenspektrometrie.

Das Verfahren gemäß der vorliegenden Erfindung dient der differentiellen Untersuchung des Proteoms von einem, zwei oder mehr Zell-, Gewebe- oder Körperflüssigkeitsproben während einer Analyse. Weiterhin können aber auch andere Protein-haltige Proben untersucht werden, so zum Beispiel Proteinfraktionen von Organellen oder anderen Kompartimenten von Zellen. Das Verfahren ist eine neuartige Alternative zur ICAT-Methode (Isotope-coded affinity tags), die oben genannt ist und vermeidet einige der Nachteile von ICAT. Es stellt eine neue alternative und komplementäre Technologie für die Proteom-Forschung dar. Das Verfahren der vorliegenden Erfindung wird im folgenden als MeCAT (Metal-chelate-complex-codedaffinity-tag) bezeichnet.

Beim MeCat-Verfahren werden Peptid/Protein-Proben mit einem MeCAT-Reagenz umgesetzt, das folgende Kerneigenschaften besitzt:
- reaktive Gruppen zur Kopplung an Proteine/Peptide oder andere Biomoleküle, im folgenden auch mit "PRG" bezeichnet;
- mindestens ein chelatbildender Komplex zur (möglichst stabilen) Komplexierung von Metallen, insbesondere isotopenarmen Metallen, im folgenden auch mit "Y" bezeichnet; und
- mindestens eine Affinitätsfunktion (z.B. Biotin) oder weitere reaktive Gruppe/n zur Kopplung an Trägermaterialien, feste Phasen oder andere Verbindungen (z.B. SH-Gruppe), im folgenden auch mit "A" bezeichnet.

Anstelle einer Markierung mit unterschiedlichen Isotopen werden die zu vergleichenden Proben mit MeCAT-Reagenzien umgesetzt, welche sich in den chelatgebundenen Metallionen unterscheiden. Anschließend erfolgt z.B. eine Affinitätsaufreinigung der markieren Biomoleküle, bspw. über Biotin - Streptavidin, oder ein "Fischen" durch spezifische chemische Umsetzung mit einem Trägermaterial mit späterer Wiederfreisetzung.

Im nächsten Schritt werden die markierten Biomoleküle durch multi-dimensionale Chromatographie getrennt und anschließend einer on-line oder off-line Differentialanalyse mit relativer Quantifizierung der Protein/Peptidmenge durch Massenspektrometrie unterzogen. Die korrespondierenden Peptide der einzelnen Proben sind je nach dem eingesetzten Metall unterschiedlich schwer markiert und können so in Verbindung mit einer Sequenzanalyse (Identifizierung) der Biomoleküle (Peptide) durch MS" den einzelnen Proben quantitativ zugeordnet werden.

Bevorzugt ist ein erfindungsgemäßes Verfahren, wobei die Spaltung der Peptide enzymatisch oder chemisch durchgeführt wird. Die Spaltung kann eine Hydrolyse unter der Verwendung von bekannten Proteasen, wie zum Beispiel Trypsin, Asp-N-Protease, Pepsin, Lys-C, Glu-C, Arg-C Proteinase, Asp-N Endopeptidase, BNPS-Skatole, Caspasen, Chymotrypsin, Clostripain, Faktor Xa, Glutamyl-Endopeptidase, GranzymB, Prolin-Endopeptidase, Proteinase K, Staphlokokken-Peptidase A, Thermolysin, Thrombin, Carboxypeptidasen und Kombinationen davon geeignet durchgeführt werden. Die chemische Spaltung kann mittels partieller Säurehydrolyse CNBr, Ameisensäure, Jodosobenzoesäure, NTCB (2-Nitro-5-thiocyanobenzosäure), Hydroxylamin und Kombinationen davon durchgeführt werden.

Weiterhin ist ein erfindungsgemäßes Verfahren bevorzugt, wobei die markierten Peptide nach der Freisetzung von dem Trägermaterial und vor ihrer massenspektrometrischen Analyse chromatographisch voneinander getrennt werden, insbesondere durch HPLC. Die chromatographische Technik wird je nach gewünschtem Auflösungsgrad gewählt.

Besonders bevorzugt ist ein erfindungsgemäßes Verfahren, das dadurch gekennzeichnet ist, daß mehrere Protein- und/oder Peptide enthaltende Proben gemeinsam analysiert werden. Dies kann insbesondere durch die unterschiedliche Markierung von verschiedenen Proben aus verschiedenen Zellmaterialien erreicht werden.

Besonders bevorzugt ist bei den erfindungsgemäßen Verfahren, daß die markierten Peptide einer anschließenden Sequenzierung unterzogen werden. Mit der Sequenzinformation der markierten Peptide können dann Datenbanken durchsucht werden, um Rückschlüsse auf das Stammprotein schließen zu können.

In einem weiteren Aspekt der vorliegenden Erfindung wird ein Verfahren zum massenspektrometrischen Nachweis der relativen Expression von Proteinen in einer Proteine enthaltenden Probe zur Verfügung gestellt, wobei das Verfahren die Schritte umfaßt von: a) Zur Verfügung stellen einer biologischen Probe, die Proteine enthält; b) Zur Verfügung stellen eines Reagenz zur Analyse von Peptiden, das die allgemeine Formel

A-Y-PRG

aufweist, worin A mindestens eine funktionelle Gruppe zur reversiblen, kovalenten oder nicht-kovalenten Bindung an ein Trägermaterial darstellt, Y eine Gruppe ist, die mindestens einen makrozyklischen Lanthanoid-Chelatkomplex umfaßt, und PRG eine reaktive Gruppe zur selektiven Bindung an Peptide oder andere zu analysierende Biomoleküle ist; c) Spaltung der Proteine in der Probe, um Peptide zu erzeugen; d) Kopplung der Peptide mit dem Reagenz aus Schritt b); e ) Selektion der in Schritt d) markierten Peptide unter Verwendung mindestens einer funktionelle Gruppe zur reversiblen, kovalenten oder nicht-kovalenten Bindung an ein Trägermaterial und Entfernung von nicht gebundenen Peptiden; f) Freisetzen der affinitätsgebundenen Peptide von dem Trägermaterial und Elution von der Matrix; und g) Nachweisen und Identifizieren der markierten Peptide mittels Massenspektrometrie, und h) Messen der relativen Anwesenheit der verschieden markierten Peptide als getrennte Ionenpeaks, um die relative Expression des Proteins zu bestimmen, von dem die affinitätsmarkierten Peptide abstammen. Anhand des analysierten Expressionsmusters lassen sich in bisher unerreichter Auflösung Rückschlüsse auf die verschiedenen Zustände von Zellen ziehen oder diagnostische Parameter erhalten, die von Protein-enthaltenden Probe abgeleitet sind.

In einem weiteren Verfahren gemäß der vorliegenden Erfindung kann die Anordnung der Gruppen A, X und PRG vertauscht sein. In der Tat kann das erfindungsgemäße Reagenz in seinen verschiedenen Bestandteilen verschieden orientiert vorliegen, solange wie alle funktionellen Erfordernisse zur Durchführung von MeCAT immer noch vorhanden sind.

Bevorzugterweise werden die markierten Peptide in dem erfindungsgemäßen Verfahren mittels Tandem-Techniken, wie zum Beispiel matrixassistierte Laserdesorptions/Ionisations (MALDI) time-of-flight (TOF)-TOF-MS und die Elektrospray-ionisations-(ESI)-MS nachgewiesen. Dabei kann ein interner Standard bei der Analyse verwendet werden, der in die Probe eingefügt werden kann.

Die Erfindung umfaßt sowohl das MeCAT-Verfahren als auch die Synthese der neuartigen MeCAT-Verbindungen. So wird gemäß einem weiteren Aspekt der vorliegenden Erfindung ein Reagenz für die massenspektroskopische Multiplex-Analyse von Peptiden zur Verfügung gestellt, das die allgemeine Formel:

A-Y-PRG

aufweist, worin A mindestens eine funktionelle Gruppe zur reversiblen, kovalenten oder nicht-kovalenten Bindung an ein Trägermaterial darstellt, Y eine Gruppe ist, die mindestens einen makrozyklischen Lanthanoid-Chelatkomplex umfaßt, und PRG eine reaktive Gruppe zur selektiven Bindung von Peptiden oder anderen zu analysierenden Biomolekülen ist, die analysiert werden sollen.

In einem alternativen Reagenz gemäß der vorliegenden Erfindung kann die Anordnung der Gruppen A, X und PRG vertauscht sein. In der Tat kann das erfindungsgemäße Reagenz in seinen verschiedenen Bestandteilen verschieden orientiert vorliegen, solange wie alle funktionellen Erfordernisse zur Durchführung von MeCAT immer noch erfüllt sind.

Bevorzugterweise ist die Funktion PRG ausgewählt aus einer Sulfhydryl-reaktiven Gruppe, einer Amin-reaktiven Gruppe und einem Enzymsubstrat. Weiter bevorzugt ist, daß PRG ausgewählt ist aus der Gruppe von einer Amin-reaktiven Pentafluorphenylestergruppe, einer Amin-reaktiven N-Hydroxysuccinimidestergruppe, Sulfonylhalid, Isocyanat, Isothiocyanat, aktivem Ester, Tetrafluorphenylester, einem Säurehalid und einem Säureanhydrid, einer Homoserin Lacton-reaktiven primären Amingruppe, und einem Carboxylsäure-reaktiven Amin, Alkohol oder 2,3,5,6-Tetrafluorphenyltrifluoracetat, einer Jodacetylamidgruppe, einem Epoxid, einer α-Haloacylgruppe, einem Nitril, einem sulfonierten Alkyl, einem Arylthiol und einem Maleimid.

Besonders bevorzugt ist ein erfindungsgemäßes Reagenz, worin A ausgewählt ist aus Biotin oder modifiziertem Biotin, einem 1,2-Diol, Glutathion, Maltose, einer Nitrilotriessigsäuregruppe, einem Oligohistidin oder einem Hapten. Im Falle von Biotin kann so zum Beispiel das Reagenz an eine Streptavidin-Gruppe angekoppelt werden, um so bequem isoliert werden zu können. Besonders bevorzugt ist dabei die Verwendung einer Streptavidin-markierten Säulenmatrix oder beschichtete Perlen ("beads").

In einer weiteren Ausführungsform ist A eine an ein Trägermaterial gekoppelte reaktive Gruppe, die wieder vom Trägermaterial abgespalten werden kann. In Frage kommen u.a. Disulfid-Bindungen (S-S), die wieder reduziert und damit gespalten werden können, oder photoempfindliche Bindungen, die durch Lichteinwirkung gespalten werden können.

In einer weiteren Ausführungsform eines erfindungsgemäßen Reagenz gemäß der vorliegenden Erfindung umfaßt dieser einen chemisch und/oder enzymatisch spaltbaren Linker zwischen den Gruppen A, X und/oder PRG. Im allgemeinen kann dieser Linker aus CH₂-Gruppen aufgebaut sein, die sich zwischen den Gruppen A, X und/oder PRG befinden und diese miteinander verbinden. Eine oder mehrere der CH₂-Gruppen kann substituiert sein, wobei der Charakter der Substitutionen nicht relevant ist, solange wie die Funktionen der Gruppen A, Y und PRG nicht beeinflußt werden. Vorteilhafterweise können über die Linker jedoch andere Funktionen eingefügt werden, wie zum Beispiel die oben genannte chemische und/oder enzymatische Spaltbarkeit. Mögliche Substitutionen sind Alkyl-, Alkenyl- und Alkoxy-Gruppen, Arylgruppen, die mit einer oder mehrerer Alkyl-, Alkenyl- und AlkoxyGruppen, Arylgruppen substituiert sind, saure Gruppen und basische Gruppen. Weiterhin können Doppel- und Dreifachbindungen im Linker vorhanden sein und Heteroatome, wie zum Beispiel O, S und N, eingefügt sein, wie zum Beispiel in Form eines Linker, der eine Disulfidgruppe enthält.

Eine wesentliche Funktion des Reagenz gemäß der vorliegenden Erfindung ist dessen chelatbildende Funktion. Bei bevorzugten Reagenzien gemäß der vorliegenden ist Y ausgewählt aus einem makrozyklischen Lanthanoid-Chelatkomplex, einem funktionalisierten Tetraazamacrozyklus, einer Polyazapolyessigsäure, DOTA, einem DOTA-Derivat, NOTA, einem NOTA-Derivat, EDTA, DTPA-BP, DTPA, DO3A, HP-DO3A und DTPA-BMA. Besonders bevorzugte Verbindungen sind 1,4,7,10,13,16,19,22-Oktaazacyclotetracosan-1,4,7,10,13,16,19,22-oktaessigsäure (OTEC) und 1,4,7,10,14-17,20,23-Oktaazacyclohexacosan-1,4,7,10,14,17,20,23-oktaessigsäure (OHEC).

Die Metalle, die mit der chelatbildende Funktion des Reagenz gebunden werden können, können aus einer Vielzahl von Metallen ausgewählt werden, was die Flexibilität bei der Verwendung des Reagenz gemäß der vorliegenden Erfindung entscheidend verbessert. So kann das durch den Chelatkomplex gebundene Metall ausgewählt sein aus Ce, Dy, Er, Eu, Gd, Ho, La, Lu, Nd, Pr, Sm, Tb, Tm, und Yb. Erfindungsgemäß kann die chelatbildende Gruppe mit mehreren verschiedenen Lanthaniden markiert sein.

Ein anderer Aspekt der vorliegenden Erfindung betrifft die Verwendung des erfindungsgemäßen Reagenz zum Nachweis von Peptiden in einer biologischen Probe und/oder der relativen Expression von Proteinen in einer Proteine enthaltenden Probe. Dabei kann es sich bei der biologischen Probe um eine direkt entnommene oder vorfraktionerte Probe zur differentiellen Untersuchung des Proteoms von einem, zwei oder mehr Zell-, Gewebe- oder Körperflüssigkeitsproben handeln. Weiterhin können aber auch andere Protein-haltige Proben untersucht werden, so zum Beispiel Proteinfraktionen von Organellen oder anderen Kompartimenten von Zellen. Das Verfahren wird bevorzugt im Zuge einer Diagnose oder die Überwachung von Erkrankungen eines Tieres, insbesondere des Menschen, durch Nachweis der relativen Expression von Proteinen in einer von dem Tier entnommenen und Proteine enthaltenden Probe angewendet. Durch die Analyse und Aufdeckung von differentiell exprimierten Proteinen können Rückschlüsse auf Proteine erhalten werden, die bei Erkrankungen auf zellulärer Ebene eine Rolle spielen und die als "Targets" für therapeutische Substanzen dienen können oder für die Diagnose und Überwachung einer Therapie fungieren können.

Ein weiterer Aspekt der vorliegenden Erfindung betrifft ein Analysesatz (Kit) zur Diagnose, der mindestens ein Reagenz gemäß der vorliegenden Erfindung zusammen mit weiteren zum Nachweis von Peptiden in einer biologischen Probe und/oder der relativen Expression von Proteinen in einer Proteine enthaltenden Probe geeigneten Substanzen und/oder Enzymen, insbesondere einen internen Standard, enthält. Mittels dieses Kits kann z.B. eine Proteommarkierung durchgeführt werden, deren Produkte dann an ein zentrales Analyselabor zur massenspektrometrischen Analyse geschickt werden können.

In einer weiteren Variante des erfindungsgemäßen Verfahrens ist der Einsatz radioaktiver Metallionen denkbar, der eine besonders empfindliche Detektion, z.B. durch Szintillationsmessung ermöglicht. Die entsprechenden Ionen sind dem Fachmann im Gebiet der Radiochemie bestens bekannt und können aus jedem herkömmlichen Lehrbuch der Chemie, wie zum Beispiel dem Römpp-Lexikon Chemie, 10. Auflage, Thieme Verlag, Stuttgart, entnommen werden.

Aus der Sicht des Chemikers sind durch das ICAT-Verfahren längst nicht alle Möglichkeiten der schnellen quantitativen Protein- bzw. Proteinfunktionsanalyse ausgeschöpft. Der Kern dieser hier vorgestellten Reagenzienklasse ist die geschickte Kombination dreier verschiedener Funktionen in einem Molekül; i) die Möglichkeit, spezifisch an ein Protein zu binden, ii) die Möglichkeit einer einfachen Trennung der markierten von den unmarkierten Peptiden nach der enzymatischen oder chemischen Hydrolyse, und iii) die Möglichkeit einer relativen Quantifizierung von Peptidpaaren, die aus verschiedenen Proben (z.B. aus Zellen einer Spezies in verschiedenen Entwicklungsphasen) stammen, über die Massendifferenz korrespondierender Peptide im Massenspektrum.

Die ersten beiden Funktionen werden in vielen gängigen, analytischen Trennmethoden eingesetzt. Die dritte Funktion ist an die modernsten MS-Technologien in Verbindung mit neuesten rechnergestützten Datenbanksuchprogrammen geknüpft, die die Identifizierung eines Proteins anhand der Aminosäuresequenz eines einzigen oder einiger Peptide (beispielsweise cystein-haltige Peptide) möglich machen.

Die Vorteile der Methode liegen auf der Hand: Jede beliebige Menge an Startmaterial kann bearbeitet werden. Auch nur in geringen Mengen auftretende Proteine sind nachweisbar und quantifizierbar, da diese durch eine Cystein-spezifische Selektion angereichert werden. Durch andere, aminosäurespezifische oder substratspezifische funktionelle Gruppen im MeCAT-Reagenz können weitere Proteine in der Analyse sicher erfaßt werden. Die Komplexität des Peptidgemisches wird dadurch reduziert, was einen wesentlich geringeren Arbeitsaufwand und eine schnellere und erfolgreiche Proteinidentifizierung durch Datenbanksuchprogramme mit sich bringt.

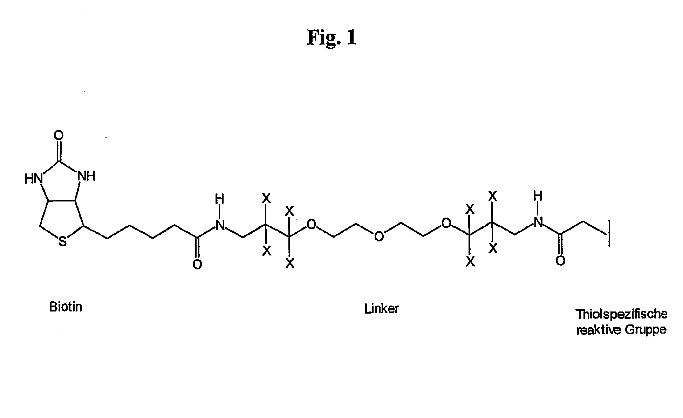

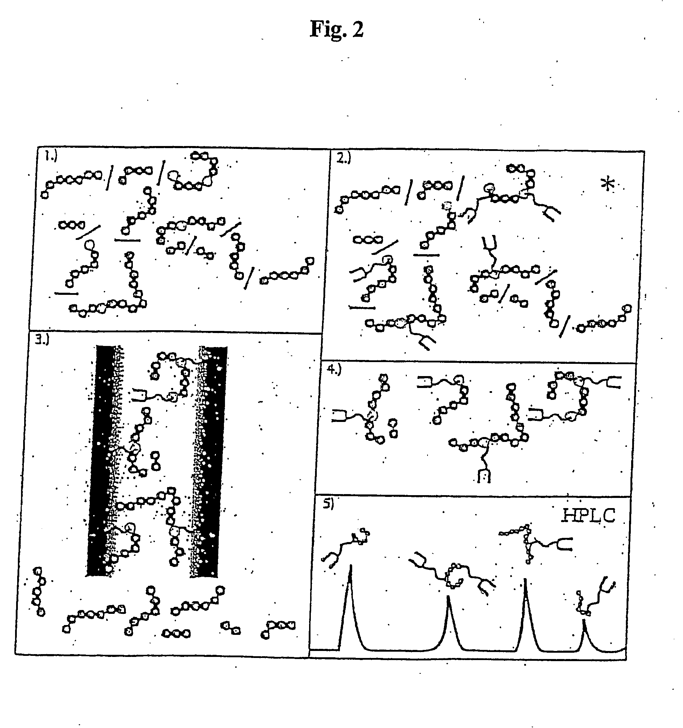

Anstelle einer Isotopenmarkierung stellt die vorliegende Erfindung den Einbau eines Metallchelatkomplexes in das Reagenz als Alternative zur Verfügung. Ein Konzept, wie diese Reagenzien aufgebaut sein können/ist in den Abb. 1 und 2 veranschaulicht.

Die Synthese eines isotopenmarkierten Linkers ist sehr teuer und nicht ohne weiteres möglich, denn es steht bekanntlich nur eine sehr begrenzte Auswahl stabiler Isotopenreagenzien, die ²H, ¹³C und ¹⁵N zur Verfügung. Das bedeutet z.B., daß Proben aus einer sehr begrenzten Anzahl Zellkulturen, die unterschiedlichen Bedingungen ausgesetzt wurden, auf den quantitativen und qualitativen Nachweis dynamischer Veränderungen in der Proteinproduktion hin untersucht und verglichen werden könnten. In der Literatur sind Beispiele für den Vergleich zweier Proben mit ¹H- und ²H-markierten ICATs bekannt.

Metallionen sind in wesentlich größerer Vielfalt und billiger verfügbar. Es kommt nur darauf an, geeignete zu finden und sie geschickt im aminosäurespezifischen Reagenz zu verpacken, ohne daß sie durch Dissoziations- oder Austauschreaktionen verlorengehen können.

Der in Frage kommende Chelatligand muß das Metallion so gut stabilisieren, daß der Komplex während des gesamten Prozesses vollständig intakt bleibt, seine Stabilität auch bei größeren pH-Änderungen garantiert ist und keine Austauschprozesse mit den Peptiden als potentielle Liganden stattfinden können. Die Löslichkeitseigenschaften des Komplexes dürfen sich von denen der anderen Reagenzkomponenten, d.h. der proteinreaktiven funktionellen Gruppe und des Molekülteils für chromatographische Trennzwecke, nicht grundlegend unterscheiden. Das gesamte Molekül muß vorzugsweise in der Probenflüssigkeit löslich sein, um eine effiziente Wechselwirkung des Tags mit den spezifischen Proteinbindungsstellen zu gewährleisten.

Für eine schnelle und eindeutige Proteinidentifizierung kann man in das proteinreaktive Reagenz ein Metallion einfügen, das man in Proteinen normalerweise nicht findet und das ein sehr charakteristisches Isotopenmuster aufweist. Dieses wird man sehr leicht im Massenspektrum des markierten Peptids wiederfinden. Computeralgorithmen können automatisch das experimentell beobachtete Isotopenmuster des Massefragments vergleichen, mit oder ohne die metallionen- bzw. massespezifische Markierung. Dies erfordert keine höheren Ansprüche an die Empfindlichkeit des verwendeten Massenspektrometers. Die an Peptide gebundenen Komplexbildner werden im Gegenteil die Nachweisempfindlichkeit positiv beeinflußen, da normalerweise in der Massenspektrometrie von Peptiden Komplexbildner als starke Kontaminanten bekannt sind und deshalb schon in geringsten Konzentrationen vermieden werden sollten. Über ein automatisches Screening der Massenspektren aller über 2DLC oder eine andere geeignete Methode getrennten Peptide sollte es möglich sein, in einem Peptidgemisch, welches vorwiegend Peptide ohne Cysteinreste enthält, die Cystein-haltigen Peptide aufgrund des speziellen Isotopenmusters sehr schnell und eindeutig zu selektieren. Und nur die genau bestimmte Masse dieser selektierten Peptide wird zur Proteinidentifizierung durch Korrelation der experimentellen Daten mit denen von Genom- bzw. Proteindatenbanken herangezogen. Eine Sequenzierung der Peptide durch CID-MS ermöglicht die Identifikation.

Für eine relative Proteinquantifizierung und -qualifizierung in mehreren Proteinproben kommen mehrere Metallchelatkomplexe mit identischem Ligandteil, aber mit unterschiedlichen Metallionen in Betracht, die sich in ihrer thermodynamischen Stabilität und in ihrem kinetischen Verhalten so ähnlich sein müssen, daß Metallaustauschprozessse untereinander auszuschließen sind. Die relativen Atomgewichte der Metallionen sollten sich um nicht mehr als 10 Dalton unterscheiden, um im Massenspektrum zusammengehörige Peptidpaare leicht ausfindig machen zu können. Die Metallionen sollten weiterhin isotopenarm sein, um eine Zuordnung nicht unnötig zu erschweren. Neben der proteinreaktiven funktionellen Gruppe kann das metallionenspezifische Reagenz eine Molekülkomponente zur säulenchromatographischen Abtrennung der markierten Peptide nach der Hydrolyse des Proteins besitzen. In Abb.1 ist die bevorzugte Strategie zur Quantifizierung der Proteinexpression mit Hilfe von metallspezifisch markierten Reagenzien (MeCATs/MeCODs) schematisch dargestellt.

Um das Metallion effizient zu binden, eignen sich als Chelate makrocyclische Liganden besonders gut, da sie sich durch eine hohe thermodynamische Stabilität und kinetische Inertheit hinsichtlich Dissoziation auszeichnen. Auf Grund ihrer topologischen Besonderheiten besitzen Makrocyclen eine Vielzahl strategisch verteilter Donoratome, die bei geeigneter Konformation und Größe des Liganden in effektiver Weise mit dem Metallion wcchselwirken können. Eine "statistische Stabilisierung" folgt aus der sehr geringen Wahrscheinlichkeit für einen gleichzeitigen Bruch aller Metall-Ligand-Donorbindungen. Ähnlich wie bei den Rezeptorbindungsstellen von Enzymen bewirken viele, im einzelnen nur schwache koordinative Wechselwirkungen bei geeigneter molekularer Architektur eine nicht nur stabile, sondern auch selektive Bindung des Metallions. Dadurch wird, im Gegensatz zu offenkettigen Liganden der Austausch mit biologisch relevanten Metallionen wirksam unterbunden (siehe Tabelle 1).

Die vorliegende Erfindung betrifft ein Verfahren und ein zu dessen Durchführung geeignetes Reagenz, das eine reproduzierbare systematische, qualitative und quantitative Proteomcharakterisierung mit Hilfe von nicht-Isotopen metallkodierten Markern und u.a. modernsten massenspektrometrischen Tandem-Methoden beinhaltet.

Der Metallcode ist ein makrocyclischer Lanthanoidchelatkomplex auf der Basis von DOTA (1,4,7,10-Tetraazcyclododecan-1,4,7,10-Tetraessigsäure) oder ein Übergangsmetallkomplex auf der Basis von NOTA (1,4,7-Triazacyclononan-1,4,7-Triessigsäure), der mit einer aminosäurespezifischen funktionellen Gruppe und einer weiteren Molekülkomponente zur chromatographischen Trennung der markierten Peptide ausgestattet sein muß. Der zu synthetisierende Marker muß sich durch gute Wasserlöslichkeit und hohe kinetische Stabilität auszeichnen. Verbindungen mit verschiedenen Lanthanoidionen dürfen sich hinsichtlich ihrer chemischen Reaktivität und physikalischen Eigenschaften nicht signifikant unterscheiden. Die metallcodierten Marker werden hinsichtlich ihrer Struktur, ihrer thermodynamischen Eigenschaften in wäßriger Lösung und ihres Reaktionsverhaltens gegenüber Peptiden charakterisiert. Ihre reproduzierbare Anwendbarkeit in der Proteomanalyse muß an Modellversuchen und Realproben in Verbindung mit mehrdimensionaler Chromatographie, MS/MS und Datenbankanalyse getestet werden.

Die metallkodierten Marker werden "site-spezifisch" kovalent an die Proteine von Zellysaten gebunden. Im Anschluß an die Proteolyse der Proteine werden die metallmarkierten Peptide chromatographisch isoliert und weiter aufgetrennt, um dann massenspektrometrisch quantifiziert und im zweiten Schritt sequenziert zu werden. Über einen direkten quantitativen Vergleich wohl determinierter Zustände werden Differenzen in der Proteinzusammensetzung herausgestellt, welche schließlich mit biologischen Auswirkungen korreliert werden müssen.

In Verbindung mit einer Datenbanksuche ist es möglich, anhand eines oder nur weniger Peptide die gefragten Stammproteine zu identifizieren.

Auf diesem Gebiet der Koordinationschemie gibt es zahlreiche Arbeiten aus den letzten 15-20 Jahren, auf deren Offenbarung sich im Rahmen der vorliegenden Erfindung ohne weiteres zurückgreifen läßt (siehe Tabelle 1).

Der cyclische Ligand, der vorzugsweise ein funktionalisierter Tetraazamakrocyclus, d.h. ein DOTA-Derivat (1,4,7,10-Tetraazcyclododecan-1,4,7,10-Tetraessigsäure) oder ein Triazamakrocyclus, ein NOTA-Derivat (NOTA = 1,4,7-Triazacyclononan-1,4,7-Triessigsäure) sein kann, wird entweder aus Aminosäuren aufgebaut oder nach in der Arbeitsgruppe der Anmelder unlängst entwickelten, sehr effizienten, templatgestützten Cyclisierungsreaktionen. Die proteinreaktive Gruppe und die Funktion zur Peptidisolierung (z.B. eine spezielle Aminosäure zur kovalenten Bindung an eine Säule, die Isothiocyanatgruppen enthält oder Biotin zur Affinitätschromatographie) können in das Kohlenstoffgerüst des makrocyclischen Liganden eingebaut sein, oder der Metallchelatkomplex wird über einen Linker mit der proteinreaktiven Gruppe und der Funktion zur Peptidisolierung geeignet verknüpft.

Als Metallionen für den NOTA-Liganden eignen sich Übergangsmetallionen wie Kupfer(II), Nickel(II) und Zink(II).

Für DOTA-artige Liganden bieten sich die Lanthanoidionen an, die sehr stabile Komplexe mit vergleichbar hohen Komplexstabilitätskonstanten und sehr ähnlichen Molekulargewichten mit dieser Ligandklasse bilden (siehe Tabelle 2). Sie gleichen sich sehr stark in ihren chemischen Eigenschaften, und die Kontraktion des Ionenradius infolge der Massenzunahme hat im Fall der sehr gut untersuchten Lanthanoid-DOTA-Komplexe (DOTA = 1,4,7,10-Tetraazacyclododecan-1,4,7,10-Tetraessigsäure) nur einen verschwindend geringen Einfluß auf die kinetische Stabilität von Lanthanoidchelatkomplexen. Die hohe *in vivo* Stabilität dieser Verbindungen führte zur erfolgreichen Anwendung des DOTA-Gadolimum(III)-Komplexes als Kontrastmittel in der Magnetresonanztomographie. Für *in vivo* Anwendungen ist die kinetische Stabilität des Komplexes weit wichtiger als ihre thermodynamische Stabilität. Ein inerter Komplex geht keine Ligand- oder Metallaustauschreaktionen ein, selbst wenn die thermodynamische Stabilitätskonstante nicht sehr hoch ist. Ein Grund dafür, daß DOTA-Lanthanoid-Komplexe sowohl sehr stabil als auch inert sind, ist das optimale Größenverhältnis zwischen dem Metallion und dem vom Liganden zur Verfügung gestellten Hohlraum. Metallion und Ligand sind ein starres, gut abgeschlossenes Gebilde, das unter physiologischen Bedingungen extrem langsam dissoziiert und erst im sauren Medium durch Protonen angreifbar ist. [Gd(DOTA)(H₂O)]⁻ besitzt eine Halbwertszeit von 200 Tagen in wäßriger Lösung bei einem p[H] von 5 und von 85 Tagen bei einem p[H] von 2. Die gut untersuchte Metallaustauschreaktion zwischen [Gd(DOTA)]⁻ und [Eu(DOTA)]⁻ im pH-Bereich 3,2-5,0 zeigt, daß der geschwindigkeitsbestimmende Schritt dieser Austauschreaktion die protonenassistierte Dissoziation von [Gd(DOTA)]⁻ ist. Selbst wenn eine Protonierung an den Acetatgruppen stattfindet, sind diese mono- und diprotonierten Komplexe nicht reaktiv, da sich das Metallion innerhalb des Koordinationskäfigs befindet. Um diesen zu zerstören, müssen die Protonen zu den N-Atomen transferiert werden. Dieser Prozeß geht nur sehr langsam über ein Rearrangement des gesamten Komplexes vonstatten. Auf der Basis dieser Untersuchung sind Metallaustauschreaktionen zwischen DOTA-Lanthanoid-Komplexen im relevanten pH- und Zeitbereich mit sehr hoher Wahrscheinlichkeit auszuschließen.

**Tabelle 1. Stabilitatskonstanten, LD₅₀-Rate und Selektivitätsfaktor (Kₛₑₗ) für ausgewählte Liganden.**

| **Ligand** | **LD₅₀^{a}** | **Log** | **Log** | **Log** | **Log** | **Log** |
|---|---|---|---|---|---|---|
| | | **Kₛₑₗ** | **K_{GdL}** | **K_{Cal}** | **K_{CuL}** | **K_{ZnL}** |
| EDTA | 0,3 | 4,23 | 17,7 | 10,61 | 18,78 | 16,5 |
| DTPA-BP | 2,8 | 5,32 | 16,83 | | | |
| DTPA | 5,6 | 7,04 | 22,46 | 10,75 | 21,38 | 18,29 |
| DOTA | 11 | 8,3 | 24,6 | 17,23 | 22,63 | 21,05 |
| DO3A | 7-9 | 4,13 | 21,0 | 11,74 | 22,87 | 19,26 |
| HP-DO3A | 12 | 6,95 | 23,8 | 14,83 | 22,84 | 19,37 |
| DTPA-BMA | 17,8 | 9,04 | 16,85 | 7,17 | 13,03 | 12,04 |

| | | | | | | |
|---|---|---|---|---|---|---|
| a) Intravenöse LD₅₀-Rate in Mäusen, mmol/kg | | | | | | |

**Tabelle 2: Stabilitätskonstanten (logK) von LnDOTA-Komplexen**

| | Relative Atom- Log Masse [g/Mol] | K_{LnDOTA} Log 1 M NaCl, 37° | K_{LnDOTA} Log 0,1MKC1,25° | K_{LnDOTA}, andere Arbeiten |
|---|---|---|---|---|
| La | 138,91 | 20,7 | 22,9 | 21,7 (0,1M KCL, 25°) |
| Ce | 140,12 | 21,6 | 23,4 | |
| Pr | 140,91 | 22,4 | 23,0 | |
| Nd | 144,24 | 22,5 | 23,0 | |
| Sm | 150,36 | 23,3 | 23,0 | |
| Eu | 151,97 | 23,7 | 23,5 | 28,2 (1M NaCl, 20°C) |
| Gd | 157,25 | 23,6 | 24,7 | 22,1 (1M NaCl, 25°C); 24,0 (0,1 M KCL, 25°C) |
| Tb | 158,93 | 23,6 | 24,7 | 28,6 (1M NaCl, 20°C) |
| Dy | 162,50 | 23,5 | 24,8 | |
| Ho | 164,93 | 23,5 | 24,5 | |
| Er | 167,26 | 23,5 | 24,4 | |
| Tm | 168,93 | 23,7 | 24,4 | |
| Yb | 173,04 | 24,0 | 25,0 | |
| Lu | 174,97 | 23,5 | 25,4 | 29,2 (1M NaCl, 25°C) |

Die Entwicklung von makrocyclischen lanthanoidspezifischen Liganden erhielt seit dem Beginn der achtziger Jahre einen beachtlichen Aufschwung durch deren erfolgreiche medizinische Anwendung sowohl in der Therapie als auch in der Diagnostik. Ein von Lauffer et al. 1999 erschienenes Review über Gd(III)-Chelate als Kontrastmittel für die Magnetresonanztomographie (MRT) gibt in eindrucksvoller Weise eine Zusammenfassung über die wichtigsten Forschungsergebnisse des letzten Jahrzehnts wieder.

Ein wichtiger Aspekt der vorliegenden Erfindung ist die Synthese, Charakterisierung und anwendungsbezogene Untersuchung von dinuklearen makrocyclischen Lanthanoidchelatkomplexen, die als potentielle MRI-Kontrastmittel für die medizinische Diagnostik konzipiert wurden. Im Gegensatz zu den sehr gut untersuchten Lanthanoidkomplexen mit dem Liganden DOTA (1,4,7,10-tetraazacyclododecan-1,4,7,10-tetraacetate) und von DOTA abgeleiteten Verbindungen sind sehr gut wasserlösliche und wasserstabile, mehrkernige makrocyclische Lanthanoidchelatkomplexe bisher nur in sehr begrenzter Zahl bekannt.

Mit dieser Zielstellung gelang es, zwei Liganden zu synthetisieren, 1,4,7,10,13,16,19,22-Oktaazacyclotetracosan-l,4,7,10,13,16,19,22-oktaessigsäure (OTEC) und 1,4,7,10,14-17,20,23-Oktaazacyclohexacosan-1,4,7,10,14,17,20,23-oktaessigsäure (OHEC), die in der Lage sind, mono- und dinukleare Lanthanoidkomplexe zu bilden. Ihre Existenz konnte mit Hilfe der FAB-Massenspektrometrie nachgewiesen werden. Als Highlight in der Koordinationschemie gelang die Bestimmung der Festkörperstrukturen der dinuklearen Chelatkomplexe des OHEC-Liganden (Ln = Y, La, Eu, Gd, Tb, Yb, Lu), die mit Hilfe der Röntgenstrukturanalyse ermittelt wurden. Neben der Strukturinformation liefern die Röntgenanalysen Hinweise auf die Zahl der in erster Koordinationssphäre koordinierten Wassermoleküle, die einen wichtigen Beitrag zur Wirksamkeit des Kontrastmittels leisten. Wir haben festgestellt, daß die Größe des Ionenradius der Metalle die Konformation des Liganden im Komplex und somit auch die Eigenschaften als MRI-Kontrastmittel entscheidend mit beeinflußt.

Mittels dynamischer NMR Messungen haben wir die Konformationsgleichgewichte der Komplexe in Lösung untersucht. Für die Yttrium- und Europiumkomplexe von OHEC erfolgte der Nachweis der erfolgreichen Synthese zusätzlich mittels ein- und zweidimensionaler NMR-Methoden. Die mono- und dinuklearen Europiumkomplexe mit OHEC konnten polarographisch näher charakterisiert werden. Die Bestimmung der Relaxivität der Gd-Komplexe erfolgte durch NMRD-Messungen (nuclear magnetic relaxation dispersion). Wir haben Relaxivitäten ermittelt, die signifikant größer sind, als bei im klinischen Einsatz befindlichen Kontrastmitteln. Deshalb besteht die berechtigte Hoffnung, hier eine neue Klasse von potentiellen Kontrastmitteln mit verbesserten Eigenschaften für die medizinische Diagnostik in den Händen zu haben.

Die Erfindung soll nun im folgenden durch Beispiele unter bezug auf die beigefügten Zeichnungen weiter erläutert werden, ohne auf diese Beispiele begrenzt zu sein. Es zeigt
- Figur 1: den Aufbau eines bei der MeCat verwendeten beispielhaften Reagenz. X ist entweder H oder eine Chelatgruppe.
- Figur 2: die schematische Darstellung eines MeCAT-Verfahrens, mit 1) enzymatischer Spaltung; 2) Kopplung mit dem MeCAT-Reagenz; 3) Selektion der markierten Peptide; 4) Elution der selektierten Peptide; 5) Trennung der markierten Peptide; anschließend massenspektrometrische Analyse. * Hier können Probe A und B vereinigt werden.

### Beispiele

### Syntheseplanung - Vorarbeiten

Ziel der Synthese ist die Darstellung eines zweifach C-substituierten Tetraazamakrocyclus. Ausgehend von einem Amino-geschützten Lysin-Hydroxysuccinimidester (2) wird durch Umsetzung zweier Äquivalente mit einem Äquivalent Ethylendiamin das peptidisch verknüpfte Di-Lysin-Derivat (3) erhalten. Die Entschützug zweier Aminofunktionen liefert die eine Komponente für die Cyclisierungsreaktion, die Mesylierung von Ethylenglycol die andere. In einer anschließenden [1+1]-Cyclokondensation erhält man den zweifach C-substituierten Tetraazadicarbonyl-Cyklus (4). Durch Reduktion der beiden Carbonyl-Funktionen gewinnt man letztlich den Tetraazacyclus (1), der an den beiden Seitenketten noch weiter funktionalisiert werden kann.

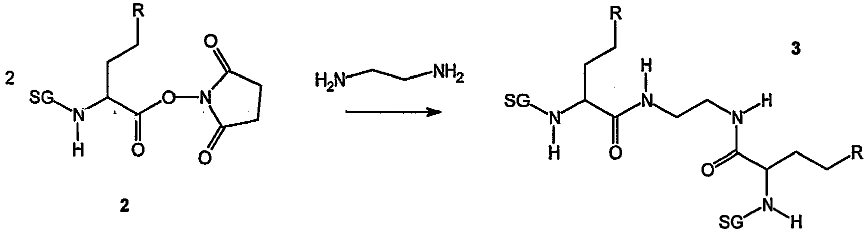

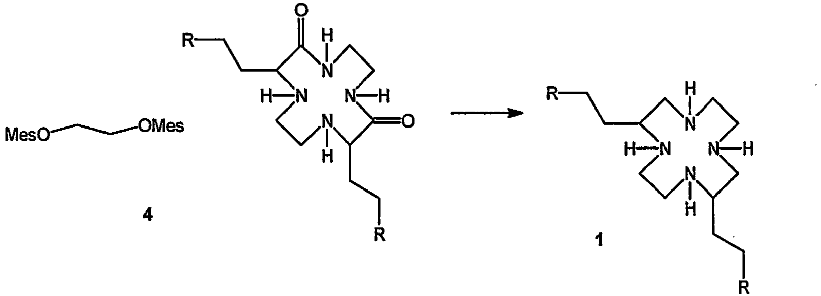

### Versuchskonzept:

Ziel dieser Versuche war die Synthese und Anwendung von neuen funktionalen Markern zur Identifizierung und Quantifizierung von Zellproteinen. Die Marker sollten 1. sich an spezifische Aminosäuregruppen denaturierter Proteine binden lassen, 2. sich mittels Affinitätschromatographie und anderer Trennmethoden aus einem großen Peptidpool isolieren lassen und 3. eine Identifizierung und Quantifizierung der Stammproteine anhand markierter Peptidfragmente mittels Massenspektrometrie und Datenbankanalyse erlauben.

Dafür galt es, folgende Bausteine in einem Molekül zu vereinen:
1. eine aminosäurespezifische oder sulhydrylspezifische Gruppe, z.B. eine Amin-reaktiven Pentafluorphenylestergruppe, eine Amin-reaktive N-Hydroxysuccinimidestergruppe, Sulfonylhalid, Isocyanat, Isothiocyanat, aktiver Ester, Tetrafluorphenylester, ein Säurehalid und ein Säureanhydrid, eine Homoserin Lacton-reaktive primäre Amingruppe, und ein Carboxylsäure-reaktives Amin, Alkohol oder 2,3,5,6-Tetrafluorphenyltrifluoracetat, eine Jodacetylamidgruppe, ein Epoxid, eine α-Haloacylgruppe, ein Nitril, ein sulfoniertes Alkyl, ein Arylthiol oder ein Maleimid, die selektiv mit einer funktionellen Gruppe im Protein, in diesem Beispiel mit SH-Gruppen im Cystein, reagiert oder eine funktionelle Gruppe, die mit einer Proteinbindungsstelle in Wechselwirkung tritt (Ligand-Rezeptor-WW),
2. Reaktive Gruppen zur Bindung an ein Trägermaterial (z.B. zur Bindung des Komplexes an ein Säulenmaterial und anschließendes Binden an Peptide) oder Biotin oder andere aus der Affinitätschromatographie bekannte Moleküle, die zwecks Abtrennung der markierten Peptide an ein entsprechendes Gegen-Reagenz gebunden wurden und im Anschluß an den Trennungsgang wieder leicht vom Restmolekül abspaltbar waren, wobei reaktive Gruppen aus Säurehalogeniden, Aldehyden, Isocyanatderivaten, Succinimid-Derivaten, Imidazolyl-Carbamat-Derivaten, Traut's Reagenz-Derivaten, Sulfonsäurechlorid-Derivaten, Oxiran-Derivaten, Imidaten, Hydrazinen, Sulfosuccinimidyl-Derivaten, Diimid-Derivaten, Maleimid-Derivaten und 7-Sulfobenzofurazan-Derivaten ausgewählt sein können.
3. das Kernstück der neuen Marker, ein Makrocyclus, der Metallkomplexe hoher kinetischer und thermodynamischer Stabilität ausbildet.

Diese Marker wurden auf ihre Eignung in der Proteomanalytik, die sich die Leistungsfähigkeit der modernen Massenspektrometrie zunutze macht, untersucht. Dazu diente ein Testgemisch aus 5-10 Proteinen sowie eine Reihe von "real life samples".

### Generelle Vorgehensweise:

Das Kernstück der vorgeschlagenen Marker sind Makrocyclen auf der Basis von Polyazapolyessigsäuren (DOTA/NOTA), deren Metallkomplexe die geforderte Stabilität aufweisen. Diese Makrocyclen galt es in ausreichender Menge zu synthetisieren und dabei mit ein bzw. zwei Bindungstellen für die weiteren Bausteine der Marker auszustatten bzw. die Makrocyclen über einen geeigneten Linker mit den übrigen MeCAT Komponenten zu verknüpfen Die bevorzugte Methode geht von Aminosäuren aus, wobei die Einführung der Me-CAT Komponenten an C-Atome des Macrocyclus erfolgt. Bei einer alternativen Methode wird der Macrocyclus mit den übrigen MeCAT Komponenten über einen geeigneten Linker verknüpft. In einer dritten Methode erfolgt die Synthese gemäß der Festphasenpeptidsynthese an einem Peptidsynthesizer und anschließendem intramolekularen Tosylamidringschluß. Die MeCAT Reagenzien waren wiederum an C-Atome des Azamacrocyclus gebunden. Die Reinigung der Peptide erfolgte mittels präparativer HPLC.

Die Liganden wurden mit dreiwertigen Lanthanoidionen komplexiert, umfassend charakterisiert und auf ihre Eignung als MeCAT Reagenzien mit den gewünschten Eigenschaften getestet. Folgende Anforderungen wurden an das Reagenz gestellt:
- Die Komplexe müssen ausreichend kinetisch stabil sein, d.h. Metallaustauschreaktionen sollten vernachlässigbar klein sein.
- Diese Kodierungstechnik mit verschiedenen Metallionen diente als interne Standardmethode, um die relative Konzentration der unterschiedlich markierten Komponenten aus verschiedenen Proben zu bestimmen. Deshalb mußten die chemischen und physikalischen Eigenschaften der MeCAT-Reagenzien mit verschiedenen Metallionen u. a. hinsichtlich der Reaktion mit den Proteinen und ihres chromatographischen Trennverhaltens möglichst weitgehend identisch sein.
- Die Molmasse sollte die des ICAT Reagenzes nicht wesentlich übersteigen.

Folgende Untersuchungen wurden dann durchgeführt:
a) Charakterisierung der MeCAT Reagenzien mittels MS und NMR;
b) Test der aminosäurespezifischen bzw. substratspezifischen Bindungseigenschaften der MeCAT-Reagenzien und des Verhaltens der markierten Peptide im Massenspektrometer anhand eines kleinen ca. 10 Peptide enthaltenden Substanzpools;
c) systematische Untersuchung und Optimierung des Verhaltens der markierten und unmarkierten Peptide in der Affinitätschromatographie und anderen chromatographischen Trennverfahren (lonenaustauschchromatographie, RP-Chromatographie), insbesondere Untersuchung der Reproduzierbarkeit und der Wiederfindungsraie der markierten Peptide
d) Nachweis der Reproduzierbarkeit des mittels geeigneten massenspektrometrischen Methoden bestimmten relativen Konzentrationsverhältnissen der mit unterschiedlichen Metallionen markierten und sonst chemisch identischen Peptide anhand des relativen Signalintensitätsverhältnisses der entsprechend zusammengehörigen Peptidpeaks im Massenspektrum
e) Untersuchung der Eigenschaften der MeCAT Reagenzien an Realproben.

### Darstellung geeigneter Linker sowie deren Verknüpfung mit den MeCat-Komponenten

a) Der Linker wurde mit einer Biotineinheit zur Bindung an Avidin verknüpft.
b) Der Linker wurde mit Glycin verknüpft zur kovalenten Bindung über Isothiocyanat- Gruppen an eine chromatographische Säule. Damit die unmarkierten Peptide die Säule ungehindert passieren können, mußten in diesem Fall zuvor sämtliche Aminogruppen mit Phenylisothiocyanat derivatisiert werden.
c) Der Linker wurde mit einer Jodessigsäureeinheit zur selektiven Markierung cysteinhaltiger Peptide verknüpft.
d) Der Linker wurde mit einer Succinsäureanhydrideinheit zur Markierung aminhaltiger Peptide verknüpft.

### Literaturverzeichnis:

- F. Sanger, Nature 1977, 265, 687.
- V. C. Wasinger, S, J. Cordwell, A. Cerpa-Poljak, J. X. Yan, A. A. Gooley, M. R. Wilkins, M. W. Duncan, R. Harris, K. L. Williams, I. Humphery-Smith, Elektrophoresis 1995,16, 1090.
- J.E. Celis, Electrophoresis 1990, 11, 989.
- K. B. Mullis, F. A. Faloona, Methods Enzymol. 1987, 155, 335.
- A. J. Link, Electrophoresis 1997,18, 1314.
- A. e. a. Shevchenko, Proc. Nail. Acad. Sei. USA 1996, 93, 1440,
- D. Figeys, Electrophoresis 1998, 19, 1811.
- D. R. e. a. Goodlett, Anal. Chem. 2000, 72, 1112.
- K. L. Williams, Electrophoresis 1999, 20, 678.
- M. Quadroni, P. James, Electrophoresis 1999, 20, 664.
- I. Humphery-Smith, S. J. Cordwell, W. P. Blackstock, Electrophoresis 1997, J8, 1217.
- R. H. Aebersold. M. H. Gelb, S. P. Gygi, C, R. Scott, F. Turecek, S. Gerber, B. Rist, in PCT/US99/19415.
- P. S. Gygi, B. Rist, S. A. Gerber, F, Turecek, M. H. Gelb, R. Aebersold, Nature Biotechnology 1999, 77, 994.
- D. F. Hunt, J. R. Yates, J, Shabanowilz, S. Winston, C. R. Hauer, Proc. Natl. Acad. Sci. USA 1986, 83, 6233.
- C. S. Spahr, S. A, Susin, E. J. Bures, J. H. Robinson, M- T. Davis, M. D, McGinley, G. Kroemer, S. D. Patterson, Electrophoresis 2000, 21, 1635.
- S. P. Gygi, B. Rist, T. J. Griffin, J. Eng, R. Aebersold, Journal of Proteome Research 2002.
- T. J. Griffin, D. K. M. Han, S. P. Gygi, B. Rist, H. Lee, R. Aebersold, K. C. Parker, Journal of the American Society for Mass Spectrometry 2001, 12, 123 8.
- B. J M. B. Smolka, H. Zhou, S. Purkayastha, R. Aebersold, Anal. Biochem. 2001, 297, 25
- D. K. Han, J. Eng, H. Zhou, R. Aebersold, Nature Biotechnology 2001, 19, 946.
- T. J. Griffin, S. P. Gygi, B. Rist, R. Aebersold, A. Loboda, A. Jilkine, W. Ens, K. G Standing, Anal. Chem. 2001, 73, 978.
- R. Zhang, F. E. Regnier, Journal of Proteome Research 2002.
- X. Wang, T. Jin, V. Comblin, A. Lopez-Mut, M- E., J. F. Desreux, Inorg. Chem. 1992, 31, 1095.
- G. R. Choppin, K. M. Schaab, Morg. Chim Acia 1996, 252, 299.
- A. E. Martell, R. J. Motekaitis, E. T. Clarke, R. Delgado, Y, Sun, R. Ma, Supramolecular Chemistry 1996, 6, 353.
- R. Delgado, J. J. R. Frausto Da Silva, Talanta 1982, 29, 815,
- E, Töth, E. Brücher, I. Lazar, I. Toth, Inorg. Chem. 1994, 33, 4070.
- P. Caravan, J. J. Ellison, T. J. McMuny, R. B. Lauffer, Chem. Rev. 1999, 99, 2293.
- W. P. Cacheris. S. C. Ouay, S. M. Rocklage, Magnetic Resonance Imaging 1990, 8, 467.

## Patentansprüche

1. Verfahren zur massenspektrometrischen Identifizierung und Quantifizierung von einem oder mehreren Proteinen in einer Probe, die ein Gemisch von Proteinen enthält, wobei das Verfahren die Schritte umfaßt von:
a) Zur Verfügung stellen einer Probe, die ein Gemisch von Proteinen enthält;
b) Zur Verfügung stellen eines Reagenz zur Analyse von Peptiden, das die allgemeine Formel
A-Y-PRG
aufweist, worin
A mindestens eine funktionelle Gruppe zur reversiblen, kovalenten oder nicht-kovalenten Bindung an ein Trägermaterial darstellt,
Y eine Gruppe ist, die mindestens einen makrozyklischen Lanthanoid-Chelatkomplex umfaßt, und
PRG eine reaktive Gruppe zur selektiven Bindung an Peptide oder andere zu analysierende Biomoleküle ist;
c) Spaltung der Proteine in der Probe, um Peptide zu erzeugen;
d) Kopplung der Peptide mit dem Reagenz aus Schritt b)
e) Selektion der in Schritt d) markierten Peptide unter der Verwendung einer funktionellen Gruppe zur reversiblen, kovalenten oder nicht-kovalenten Bindung an ein Trägermaterial und Entfernung von nicht-gebundenen Peptiden;
f) Freisetzen der gebundenen Peptide von dem Trägermaterial und Elution von der Matrix; und
g) Nachweisen und Identifizieren der markierten Peptide durch Massenspektrometrie.

2. Verfahren nach Anspruch 1, wobei die Spaltung der Peptide enzymatisch oder chemisch durchgeführt wird.

3. Verfahren nach Anspruch 1 oder 2, wobei die markierten Peptide nach der Freisetzung von dem Trägermaterial und vor ihrer massenspektrometrischen Analyse voneinander getrennt werden, insbesondere durch HPLC.

4. Verfahren nach einem der Ansprüche 1 bis 3, **dadurch gekennzeichnet, daß** mehrere Protein- und/oder Peptide enthaltende Proben gemeinsam analysiert werden.

5. Verfahren nach einem der Ansprüche 1 bis 4, weiterhin umfassend die Sequenzierung der markierten Peptide.

6. Verfahren zum massenspektrometrischen Nachweis der relativen Expression von Proteinen in einer Proteine enthaltenden Probe, wobei das Verfahren die Schritte umfaßt von
a) Zur Verfügung stellen einer biologischen Probe, die Proteine enthält;
b) Zur Verfügung stellen eines Reagenz zur Analyse von Peptiden, das die allgemeine Formel
A-Y-PRG
aufweist, worin
A mindestens eine funktionelle Gruppe zur reversiblen, kovalenten oder nicht-kovalenten Bindung an ein Trägermaterial darstellt,
Y eine Gruppe ist, die mindestens einen makrozyklischen Lanthanoid-Chelatkomplex umfaßt, und
PRG eine reaktive Gruppe zur selektiven Bindung an Peptide oder andere zu analysierende Biomoleküle ist;
c) Spaltung der Proteine in der Probe, um Peptide zu erzeugen;
d) Kopplung der Peptide mit dem Reagenz aus Schritt b)
e) Selektion der in Schritt d) markierten Peptide unter der Verwendung einer funktionellen Gruppe zur reversiblen, kovalenten oder nicht-kovalenten Bindung an ein Trägermaterial und Entfernung von nicht gebundenen Peptiden;
f) Freisetzen der gebundenen Peptide von dem Trägermaterial und Elution von der Matrix; und
g) Nachweisen und Identifizieren der markierten Peptide mittels Massenspektrometrie, und
h) Messen der relativen Anwesenheit der verschieden markierten Peptide als getrennte Ionenpeaks, um die relative Expression des Proteins zu bestimmen, von dem das affinitätsmarkierte Peptid abstammt.

7. Verfahren nach einem der Ansprüche 1 bis 6, **dadurch gekennzeichnet, daß** die Anordnung der Gruppen A, Y und PRG vertauscht ist.

8. Verfahren nach einem der Ansprüche 1 bis 7, **dadurch gekennzeichnet, daß** die markierten Peptide mittels Tandem-Techniken, wie zum Beispiel matrixassistierte Laserdesorptions/Ionisations (MALDI) time-of-flight (TOF)-TOF-MS und die Elektrospray-ionisations-(ESI)-MS nachgewiesen werden.

9. Reagenz für die massenspektroskopische Multiplex-Analyse von Peptiden, das die allgemeine Formel:
A-Y-PRG
aufweist, worin
A mindestens eine funktionelle Gruppe zur reversiblen, kovalenten oder nicht-kovalenten Bindung an ein Trägermaterial darstellt,
Y eine Gruppe ist, die mindestens einen makrozyklischen Lanthanoid-Chelatkomplex umfaßt, und
PRG eine reaktive Gruppe zur selektiven Bindung von Peptiden oder anderen zu analysierenden Biomolekülen ist, die analysiert werden sollen.

10. Reagenz nach Anspruch 9, wobei die Anordnung der Gruppen A, Y und PRG vertauscht ist.

11. Reagenz nach Anspruch 9 oder 10, worin PRG ausgewählt ist aus der Gruppe, bestehend aus einer Sulfhydryl-reaktiven Gruppe, einer Amin-reaktiven Gruppe und einem Enzymsubstrat.

12. Reagenz nach Anspruch 11, wobei PRG ausgewählt ist aus der Gruppe, bestehend aus einer Amin-reaktiven Pentafluorphenylestergruppe, einer Amin-reaktiven N-Hydroxysuccinimidestergruppe, Sulfonylhalid, Isocyanat, Isothiocyanat, aktivem Ester, Tetrafluorphenylester, einem Säurehalid und einem Säureanhydrid, einer Homoserin Lacton-reaktiven primären Amingruppe, und einem Carboxylsäure-reaktiven Amin, Alkohol oder 2,3,5,6-Tetrafluorphenyltrifluoracetat, einer Jodacetylamidgruppe, einem Epoxid, einer α-Haloacylgruppe, einem Nitril, einem sulfonierten Alkyl, einem Arylthiol und einem Maleimid.

13. Reagenz nach einem der Ansprüche 9 bis 12, worin A ausgewählt ist aus der Gruppe, bestehend aus Biotin oder modifiziertem Biotin, einem 1,2-Diol, Glutathion, Maltose, einer Nitrilotriessigsäuregruppe, einem Oligohistidin und einem Hapten oder anderen reaktiven Reagenzien, die eine reversible Bindung an ein Trägermaterial ermöglichen.

14. Reagenz nach einem der Ansprüche 9 bis 13, weiterhin umfassend einen chemisch und/oder enzymatisch und/oder durch Strahlen- oder Lichteinwirkung spaltbaren Linker zwischen den Gruppen A, Y und/oder PRG.

15. Reagenz nach Anspruch 14, wobei der Linker eine Disulfidgruppe enthält.

16. Reagenz nach einem der Ansprüche 9 bis 15, wobei das durch den Chelatkomplex gebundene Lanthanid ausgewählt ist aus Ce, Dy, Er, Eu, Gd, Ho, La, Lu, Nd, Pr, Sm, Tb, Tm und Yb.

17. Reagenz nach einem der Ansprüche 9 bis 16, wobei die chelatbildende Gruppe mit mehreren verschiedenen Lanthaniden markiert ist.

18. Verwendung eines Reagenz nach einem der Ansprüche 9 bis 16 zum Nachweis von Peptiden in einer biologischen Probe und/oder der relativen Expression von Proteinen in einer Proteine enthaltenden Probe.

19. Verwendung eines Reagenz nach einem der Ansprüche 9 bis 16 zur in-vitro Diagnose von Erkrankungen eines Tieres, insbesondere des Menschen, durch Nachweis der relativen Expression von Proteinen in einer von dem Tier entnommenen und Proteine enthaltenden Probe.

20. Diagnostischer Kit, enthaltend ein Reagenz nach einem der Ansprüche 9 bis 16 zusammen mit weiteren zum Nachweis von Peptiden in einer biologischen Probe und/oder der relativen Expression von Proteinen in einer Proteine enthaltenden Probe geeigneten Substanzen und/oder Enzymen, insbesondere einen internen Standard.

## Claims

1. Method for the mass spectrometric identification and quantification of one or more proteins in a sample containing a mixture of proteins, wherein said method comprises the steps of:
a) Providing a sample which contains a mixture of proteins;
b) Providing a reagent for the analysis of peptides which has the general formula
A-Y-PRG
in which
A constitutes at least one functional group for the reversible, covalent or non-covalent binding to a support material,
Y is a group comprising at least one macrocyclic lanthanoid chelate complex, and PRG is a reactive group for the selective binding to peptides or other biomolecules to be analyzed;
c) Cleaving the proteins in the sample in order to produce peptides;
d) Coupling the peptides to the reagent of step b);
e) Selecting the peptides labeled in step d) under the employment of a functional group for the reversible, covalent or non-covalent binding to a support material and removal of the unbound peptides;
f) Releasing the bound peptides from the support material and elution from the matrix; and
g) Detecting and identifying the labeled peptides by means of mass spectrometry.

2. Method according to claim 1, wherein the cleavage of the peptides is performed enzymatically or chemically.

3. Method according to claim 1 or 2, wherein the labeled peptides, after their release from the support material and before their analysis by mass spectrometry, are separated from each other, in particular by means of HPLC.

4. Method according to one of the claims 1 to 3, **characterized in that** several protein- and/or peptide-containing samples are analyzed together.

5. Method according to one of the claims 1 to 4, moreover comprising the sequencing of the labeled peptides.

6. Method for the mass spectrometric detection of the relative expression of proteins in a protein-containing sample, wherein said method comprises the steps of:
a) Providing a biological sample which contains proteins;
b) Providing a reagent for the analysis of peptides which has the general formula
A-Y-PRG
in which
A constitutes at least one functional group for the reversible, covalent or non-covalent binding to a support material,
Y is a group comprising at least one macrocyclic lanthanoid chelate complex, and PRG is a reactive group for the selective binding to peptides or other biomolecules to be analyzed;
c) Cleaving the proteins in the sample in order to produce peptides;
d) Coupling the peptides to the reagent of step b);
e) Selecting the peptides labeled in step d) under the employment of a functional group for the reversible, covalent or non-covalent binding to a support material and removal of the unbound peptides;
f) Releasing the bound peptides from the support material and elution from the matrix; and
g) Detecting and identifying the labeled peptides by means of mass spectrometry;
h) Measuring the relative occurrence of the differently labeled peptides as distinct peaks of ions in order to determine the relative expression of the protein, from which the affinity-labeled peptide is derived.

7. Method according to one of the claims 1 to 6, **characterized in that** the arrangement of the groups A, Y and PRG is interchanged.

8. Method according to one of the claims 1 to 7, **characterized in that** the labeled peptides are detected by means of tandem techniques, like e.g. matrix-assisted laser desorption/ionization (MALDI) time-of-flight (TOF)-TOF-MS and electrospray ionization (ESI)-MS.

9. Reagent for the mass spectroscopic multiplex analysis of peptides which has the general formula
A-Y-PRG
in which
A constitutes at least one functional group for the reversible, covalent or non-covalent binding to a support material,
Y is a group comprising at least one macrocyclic lanthanoid chelate complex, and PRG is a reactive group for the selective binding of peptides or other biomolecules to be analyzed, that shall be analyzed.

10. Reagent according to claim 9, wherein the arrangement of the groups A, Y and PRG is interchanged.

11. Reagent according to claim 9 or 10, wherein the PRG is selected from the group consisting of a sulfhydryl-reactive group, an amine-reactive group and an enzyme substrate.

12. Reagent according to claim 11, wherein the PRG is selected from the group consisting of an amine-reactive pentafluorophenyl ester group, an amine-reactive N-hydroxysuccinimide ester group, sulfonylhalide, isocyanate, isothiocyanate, active ester, tetrafluorophenyl ester, an acid halide and an acid anhydride, a homoserine lactone-reactive primary amine group and a carboxylic acid-reactive amine, alcohol or 2,3,5,6-tetrafluorophenyltrifluoro-acetate, a iodine acetylamide group, an epoxide, an α-haloacyl group, a nitrile, a sulfonated alkyl, an arylthiol and a maleimide.

13. Reagent according to one of the claims 9 to 12, wherein A is selected from the group consisting of biotin or modified biotin, a 1,2-diol, glutathione, maltose, a nitrilotriacetic acid group, an oligohistidine and a hapten or other reactive reagents allowing for a reversible binding to a support material.

14. Reagent according to one of the claims 9 to 13, moreover comprising a linker between the groups A, Y and/or PRG, which is cleavable in a chemical and/or enzymatic way and/or by exposure to radiation or light.

15. Reagent according to claim 14, wherein the linker contains a disulfide group.

16. Reagent according to one of the claims 9 to 15, wherein the lanthanide bound by the chelate complex is selected from Ce, Dy, Er, Eu, Gd, Ho, La, Lu, Nd, Pr, Sm, Tb, Tm and Yb.

17. Reagent according to one of the claims 9 to 16, wherein the chelate forming group is labeled with several different lanthanoids.

18. Use of a reagent according to one of the claims 9 to 16 for the detection of peptides in a biological sample and/or for determining the relative expression of proteins in a protein-containing sample.

19. Use of a reagent according to one of the claims 9 to 16 for the in-vitro diagnosis of diseases of an animal, in particular of the human, by detecting the relative expression of proteins in a protein-containing sample taken from the animal.

20. Diagnostic kit, containing a reagent according to one of the claims 9 to 16 together with further substances and/or enzymes suitable for the detection of peptides in a biological sample and/or the determination of the relative expression of proteins in a protein-containing sample, in particular containing an internal standard.

## Revendications

1. Procédé pour l'identification et la quantification par spectrométrie de masse d'une ou de plusieurs protéine(s) dans un échantillon contenant un mélange de protéines, où ledit procédé comprend les étapes qui consistent à :
a) fournir un échantillon qui contient un mélange de protéines ;
b) fournir un réactif pour l'analyse de peptides qui répond à la formule générale suivante :
A-Y-PRG
dans laquelle
A constitue au moins un groupe fonctionnel destiné à la liaison réversible, covalente ou non covalente à un matériau support,
Y est un groupe comprenant au moins un complexe de chélate de lanthanide macrocyclique, et
PRG est un groupe réactif destiné à la liaison sélective à des peptides ou à d'autres biomolécules à analyser ;
c) cliver les protéines dans l'échantillon afin de produire des peptides ;
d) coupler les peptides au réactif de l'étape b) ;
e) sélectionner les peptides marquées dans l'étape d) en employant un groupe fonctionnel pour la liaison réversible covalente ou non covalente à un matériau support et en éliminant les peptides non liés ;
f) libérer les peptides liés du matériau support et les éluer de la matrice ; et
g) détecter et identifier les peptides marqués au moyen d'une spectrométrie de masse.

2. Procédé selon la revendication 1, dans lequel le clivage des peptides est effectué par voie enzymatique ou chimique.

3. Procédé selon la revendication 1 ou 2, dans lequel les peptides marqués, après leur libération du matériau support et avant leur analyse par spectrométrie de masse, sont séparés l'un de l'autre, en particulier au moyen d'une Chromatographie Liquide à Haute Performance HPLC.

4. Procédé selon l'une des revendications 1 à 3, **caractérisé en ce que** plusieurs échantillons contenant des peptides et/ou des protéines sont analysés ensemble.

5. Procédé selon l'une des revendications 1 à 4, comprenant en outre le séquençage des peptides marqués.

6. Procédé pour la détection spectrométrique de masse de l'expression relative des protéines dans un échantillon contenant des protéines, dans lequel ledit procédé comprend les étapes qui consistent à :
a) fournir un échantillon biologique qui contient des protéines ;
b) fournir un réactif pour l'analyse des peptides qui répond à la formule générale suivante :
A-Y-PRG
dans laquelle
A constitue au moins un groupe fonctionnel destiné à la liaison réversible covalente ou non covalente à un matériau support,
Y est un groupe comprenant au moins un complexe de chélate de lanthanide macrocyclique, et PRG est un groupe réactif destiné à la liaison sélective à des peptides ou à d'autres biomolécules à analyser ;
c) cliver les protéines dans l'échantillon afin de produire des peptides ;
d) coupler les peptides au réactif de l'étape b) ;
e) sélectionner les peptides marquées dans l'étape d) en employant un groupe fonctionnel destiné à la liaison réversible covalente ou non covalente à un matériau support et en éliminant les peptides non liés ;
f) libérer les peptides liés du matériau support et les éluer de la matrice ; et
g) détecter et identifier les peptides marqués au moyen d'une spectrométrie de masse ;
h) mesurer l'apparition relative aux peptides marqués de manière différente en tant que pics distincts d'ions afin de déterminer l'expression relative de la protéine, à partir de laquelle le peptide marqué par affinité est dérivé.

7. Procédé selon l'une des revendications 1 à 6, **caractérisé en ce que** l'agencement des groupes A, Y et PRG est interchangeable.

8. Procédé selon l'une des revendications 1 à 7, **caractérisé en ce que** les peptides marqués sont détectés au moyen des techniques en tandem, comme par exemple, une désorption/ionisation laser assistée par matrice (MALDI), une spectrométrie de masse à temps de vol (TOF) dite TOF-MS et une spectrométrie de masse à ionisation par électronébulisation (ESI)-MS.

9. Réactif pour l'analyse multiplex spectroscopique de masse des peptides qui répond à la formule générale suivante :
A-Y-PRG
dans laquelle
A constitue au moins un groupe fonctionnel destiné à la liaison réversible covalente ou non covalente à un matériau support,
Y est un groupe comprenant au moins un complexe de chélate de lanthanide macrocyclique, et
PRG est un groupe réactif destiné à la liaison sélective à des peptides ou à d'autres biomolécules à analyser, qui doit être analysé ;

10. Réactif selon la revendication 9, dans lequel l'agencement des groupes A, Y et PRG est interchangeable.

11. Réactif selon la revendication 9 ou 10, dans lequel le PRG est choisi dans le groupe constitué d'un groupe réagissant avec les sulfhydryles, d'un groupe réagissant avec les amines et d'un substrat d'enzyme.

12. Réactif selon la revendication 11, dans lequel le PRG est choisi dans le groupe constitué d'un groupe ester de pentafluorophényle réagissant avec les amines, d'un groupe ester de N-hydroxysuccinimide réagissant avec les amines, d'halogénure de sulfonyle, d'isocyanate, d'isothiocyanate, d'ester actif, d'ester de tétrafluorophényle, d'halogénure d'acide et d'anhydride d'acide, d'un groupe amine primaire réagissant avec l'homosérine lactone et d'amine réagissant avec un acide carboxylique, d'alcool ou de 2,3,5,6-tétrafluorophényltrifluoroacétate, d'un groupe acétylamide d'iode, d'un époxyde, d'un groupe α-haloacyle, de nitrile, d'alkyle sulfoné, d'arylthiol et de maléimide.

13. Réactif selon l'une des revendications 9 à 12, dans lequel A est choisi dans le groupe constitué de biotine ou de biotine modifiée, d'un 1,2-diol, de glutathion, de maltose, d'un groupe acide nitrilotriacétique, d'une oligohistidine et d'un haptène ou d'autres agents réactifs permettant une liaison réversible à un matériau support.

14. Réactif selon l'une des revendications 9 à 13, comprenant en outre un agent de liaison entre les groupes A, Y et/ou PRG, qui est susceptible d'être clivé par voie chimique et/ou enzymatique et/ou par exposition à un rayonnement ou à une lumière.

15. Réactif selon la revendication 14, dans lequel l'agent de liaison contient un groupe disulfure.

16. Réactif selon l'une des revendications 9 à 15, dans lequel le lanthanide lié par le complexe chélate est choisi parmi Ce, Dy, Er, Eu, Gd, Ho, La, Lu, Nd, Pr, Sm, Tb, Tm et Yb.

17. Réactif selon l'une des revendications 9 à 16, dans lequel le groupe formant un chélate est marqué avec plusieurs lanthanides différents.

18. Utilisation d'un réactif selon l'une des revendications 9 à 16 pour la détection de peptides dans un échantillon biologique et/ou pour une détermination de l'expression relative de protéines dans un échantillon contenant des protéines.

19. Utilisation d'un réactif selon l'une des revendications 9 à 16 pour le diagnostic in vitro des maladies d'un animal, en particulier de l'être humain, par détection de l'expression relative de protéines dans un échantillon contenant des protéines prélevé sur l'animal.

20. Trousse de diagnostic, contenant un réactif selon 1"une des revendications 9 à 16 conjointement avec des substances et/ou des enzymes supplémentaires appropriées pour la détection de peptides dans un échantillon biologique et/ou la détermination de l'expression relative de protéines dans un échantillon contenant des protéines, en particulier contenant un étalon interne.
